# EUROPEAN PATENT SPECIFICATION

(11) **EP 3 214 168 B1**
(45) Date of publication and mention of the grant of the patent: **07.08.2019**
(21) Application number: 14904983.5
(22) Date of filing: 08.12.2014
(51) Int. Cl.: C12N 1/18, C12P 7/06, C12R 1/865

(54) **SACCHAROMYCES CEREVISIAE CAPABLE OF BEING CO-FERMENTED BY MULTIPLE CARBON SOURCES AND APPLICATION THEREOF**
ZUR CO-FERMENTIERUNG DURCH MEHRERE KOHLENSTOFFQUELLEN FÄHIGE SACCHAROMYCES CEREVISIAE UND VERWENDUNG DAVON
SACCHAROMYCES CEREVISIAE POUVANT ÊTRE COFERMENTÉ PAR DE MULTIPLES SOURCES DE CARBONE ET SON APPLICATION

(30) Priority: 30.10.2014 CN 201410605253
(43) Date of publication of application: 06.09.2017
(73) Proprietor: Jiangnan University, Wuxi, Jiangsu 214122 (CN)
(72) Inventor: XU, Yan, Wuxi Jiangsu 214122 (CN); WU, Qun, Wuxi Jiangsu 214122 (CN)
(74) Representative: dompatent von Kreisler Selting Werner - Partnerschaft von Patent- und Rechtsanwälten mbB
(86) International application number: PCT/CN2014/093260
(87) International publication number: WO 2016/065698

(56) References cited:
- WO-A1-2005/121337
- CN-A- 1 966 694
- CN-A- 102 965 291
- US-A1- 2009 226 993
- ANTONIUS J A VAN MARIS ET AL: "Alcoholic fermentation of carbon sources in biomass hydrolysates by Saccharomyces cerevisiae: current status", ANTONIE VAN LEEUWENHOEK, KLUWER ACADEMIC PUBLISHERS, DO, vol. 90, no. 4, 11 October 2006 (2006-10-11), pages 391-418, XP019446684, ISSN: 1572-9699, DOI: 10.1007/S10482-006-9085-7
- CHANG-LU WANG ET AL: "Microorganisms in Daqu: a starter culture of Chinese Maotai-flavor liquor", WORLD JOURNAL OF MICROBIOLOGY AND BIOTECHNOLOGY, KLUWER ACADEMIC PUBLISHERS, DO, vol. 24, no. 10, 24 March 2008 (2008-03-24), pages 2183-2190, XP019617150, ISSN: 1573-0972
- CHRISTOPHER J. MURAKAMI ET AL: "Composition and Acidification of the Culture Medium Influences Chronological Aging Similarly in Vineyard and Laboratory Yeast", PLOS ONE, vol. 6, no. 9, 19 September 2011 (2011-09-19), page e24530, XP55458652, DOI: 10.1371/journal.pone.0024530
- EDGARDO A ET AL: "Selection of thermotolerant yeast strains Saccharomyces cerevisiae for bioethanol production", ENZYME AND MICROBIAL TECHNOLOGY, STONEHAM, MA, US, vol. 43, no. 2, 10 March 2008 (2008-03-10) , pages 120-123, XP022698796, ISSN: 0141-0229, DOI: 10.1016/J.ENZMICTEC.2008.02.007 [retrieved on 2008-03-10]
- CAPPELLO M S ET AL: "Molecular and physiological characteristics of a grape yeast strain containing atypical genetic material", INTERNATIONAL JOURNAL OF FOOD MICROBIOLOGY, ELSEVIER BV, NL, vol. 144, no. 1, 25 August 2010 (2010-08-25), pages 72-80, XP027481264, ISSN: 0168-1605, DOI: 10.1016/J.IJFOODMICRO.2010.08.013 [retrieved on 2010-08-25]
- XIAOWEI LU ET AL: "Genomic and transcriptomic analyses of the Chinese Maotai-flavored liquor yeast MT1 revealed its unique multi-carbon co-utilization", BMC GENOMICS, vol. 16, no. 1, 15 December 2015 (2015-12-15), XP55458884, DOI: 10.1186/s12864-015-2263-0
- SHEN, YU ET AL.: 'Construction of Industrial Saccharomyces Cerevisiae Expressing Xylose-Metabolizing Genes in XI Pathway' PROGRESS IN BIOTECHNOLOGY vol. 25, no. 9, 30 September 2005, pages 69 - 73, XP008185478
- KAISA KARHUMAA ET AL.: 'Investigation ot limiting metabolic steps in the utilization ot xylose by recombinant Saccharomyces cerevisiae using metabolic engineering' YEAST vol. 22, 31 December 2005, ISSN 1097-0061 pages 359 - 368, XP002494168

## Description

### CROSS-REFERENCES AND RELATED APPLICATIONS

This application claims priority of Chinese Application No. 201410605253.1, entitled " A *Saccharomyces cerevisiae* strain with unique multi-carbon-coutilizing characteristics and its application", filed October 30, 2014.

### BACKGROUND OF THE INVENTION

### Field of the Invention

The present invention relates to the field of microbiological technologies and fermentation engineering technology, and more particularly, relates to a *Saccharomyces cerevisiae* strain with unique multi-carbon-coutilizing characteristics and its application.

### Description of the Related Art

*Saccharomyces cerevisiae* could use sugar to produce ethanol. It has been used in industrial brewing for thousands of years, including the production of various types of alcoholic beverages, such as brandy, whiskey, vodka, gold wine, rum, Chinese liquor, wine, rice wine, beer and so on. In addition, with the depletion of oil resources and the reproducibility of fuel ethanol production resources, humans have begun to produce ethanol using *S. cerevisiae.* Therefore, *S. cerevisiae* is extremely important to the development of human society. However, the application *of S. cerevisiae* has the following three problems:

First of all, whether cereals used as raw materials for the brewing of alcoholic beverage, or cellulose and starch used as raw material for the production of fuel ethanol, it is necessary to first degrade the above polysaccharides into monosaccharides, because that *S. cerevisiae* can not directly use cellulose and starch and other polymer raw materials. The degradation of cellulose and starch produce a variety species of sugar; for example, lignocellulosic biomass (such as crop stalks, chaff, bran, bagasse, trees, forestry processing waste, grass, etc.) could be hydrolysis (acid, or enzymatic) into glucose, xylose, arabinose, galactose and other oligosaccharides and other ingredients; starch raw materials can be hydrolysis into glucose, maltose, maltotriose, isomaltose, Pan sugar and other oligosaccharides and other ingredients. However, the diversity of these sugars is not conducive to the fermentation of *S. cerevisiae.* At first, *S. cerevisiae* has a glucose effect while using sugar, that is, *S. Cerevisiae* can only use glucose when other sugars coexist with glucose, it can begin to use other available sugars only when glucose is used up; secondly, only a small number of sugars, such as glucose, maltose and fructose, could be used by *S. cerevisiae* generally. The above characteristics greatly limit the use of *S. cerevisiae* on a variety of sugars, which not only resulting in reduced yield, but also resulting in a great waste of resources with reduced efficiency of carbon usage and resource utilization.

In order to solve the above problems, the researchers carried out a lot of work, including searching for yeast with multi-carbon-utilizing characteristics. However, generally, wild yeast could not use multiple carbon sources. Therefore, yeasts which could use multiple carbon sources were constructed by genetic engineering methods, such as protoplast fusion, foreign gene introduction or key gene knockout. Studies had reported that the recombinant *S*. *cerevisiae* constructed by genetic engineering technology could co-ferment glucose, xylose, mannose, arabinose and so on. WO 2005/121337 describes Saccharomyces strains capable of using multiple sugars such as xylose. However, the sugar species which could be used was still limited , due to the fact that the gene conducted into genetic engineering bacteria was limited; in addition, the instability is the biggest obstacle to the use of genetic engineering bacteria because *S*. *cerevisiae* is a single diploid coexistence strain; The use of genetic modification is not recognized in the brewing of alcoholic beverages.

Second, the environment of brewing and fuel ethanol fermentation process is extremely complex. For the production of fuel ethanol, only the *S. cerevisiae* wtih characteristic of tolerance to high ethanol concentrations could ferment ethanol more efficient; For the brewing process, *S. cerevisiae* not only should have characteristic of tolerance to high ethanol concentrations, but also should have characteristics of tolerance to acidity and high temperature. Therefore, only the *S. cerevisiae* with the ability to withstand the above stress could play an efficient fermentation capacity. However, conventionally used *S. cerevisiae* can only tolerate 8% ethanol, 37°C and pH 3.0.

Third, for wine brewing, *S. cerevisiae* not only should have efficient fermentation capacity, but also should have characteristics of good flavor metabolism, which making a alcoholic beverages with a more diverse flavor composition.

The above characteristics put forward three requirements for the *S. cerevisiae* used in the beverage and fuel ethanol industries. However, due to the limited technology and the defects of the wild yeast itself, it is still necessary to make important breakthroughs in the strains.

### DETAILED DESCRIPTION

The present invention provides a *S*. *cerevisiae* strain with unique multi-carbon-coutilizing characteristics and its application. The *S. cerevisiae* was screened from the Chinese Maotai-flavored liquor making environment, and it has characteristics of multi-carbon-coutilizing, tolerance to high temperature, tolerance to high concentrations of acidity, tolerance to high ethanol concentrations, good flavor metabolism. The *S. cerevisiae* could be applied in alcoholic beverages brewing, fuel ethanol production and other fields.

The first goal of the present invention is to provide a *S*. *cerevisiae,* it was deposited in China Center for Type Culture Collection (CCTCC), Wuhan University, Wuhan, Hubei Province, China, on October 10, 2014, with the accession number CCTCC M 2014463.

Said *S. cerevisiae* was screened from the Chinese Maotai-flavored liquor making environment, and is identified as a *S*. *cerevisiae* by the 26s rDNA sequence shown in SEQ ID NO. 1. The sequence was blasting on NCBI and it has 99% similarity with the 26s rDNA sequences of existed *S. cerevisiaes.*

The colony of said *S. cerevisiae* grown on YPD medium is white, round, and the edges are neat and the surface is smooth.

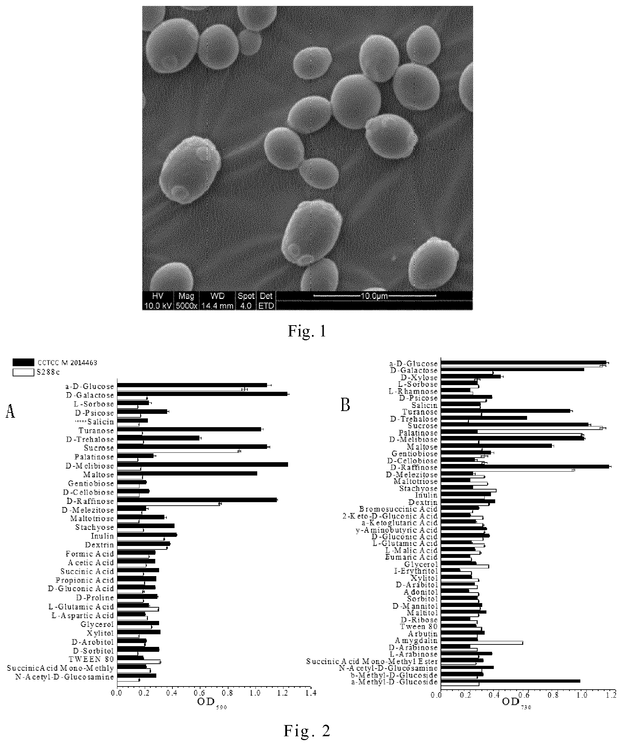

The morphology of said *S. cerevisiae* under the electron microscope is shown in Fig. 1, and it can be seen that the cells of *S. cerevisiae* are spherical or ellipsoidal, and the apical buds indicate that the breeding method is budding reproduction.

Said *S. cerevisiae* can use any of the following carbon sources: glucose, galactose, xylose, alfa ketone, sucrose, maltose, honey disaccharide, loose sugar, trehalose, raffinose and other oligosaccharides.

Said *S. cerevisiae* can also use any of mixed carbon sources with the following two or more sources: glucose, galactose, xylose, alfa sugar, sucrose, maltose, melibiose, loose sugar, trehalose, raffinose and other oligosaccharides.

Said *S. cerevisiae* can produce ethanol using one or more of the above mixed carbon sources.

Said *S. cerevisiae* could endure high temperature (44 °C), acid (pH 2.0), alcohol (16%).

Said *S. cerevisiae* is capable of producing a variety of flavor metabolites, including acids, alcohols and esters. Said acids contains hexanoic, octanoic acid, nonanoic acid, 9-decenoic acid; Said alcohols contains ethanol, isobutanol, isoamyl alcohol, butanol, hexanol, 2-nonen-1-ol, 3-methyl-1-hexanol, 1-octanol, nonanol, 3-methyl-1-hexanol, phenylethanol, lauryl alcohol, trans-neryl terephthalate, farnesol; Said esters contains ethyl acetate, isoamyl acetate, ethyl hexanoate, ethyl 4-hexenoate, ethyl octanoate, ethyl decanoate, 2-phenylethyl acetate, benzene 2-phenylethyl acetate, isohexyl-2-phenylethyl acetate, ethyl 2,4-hexadienoate, phenylacetaldehyde and acetophenone 4-vinylxaphenols.

The second goal of the present invention is to provide the application method of said *S. cerevisiae.*

Said application method was directly using *S. cerevisiae* in the production of alcoholic beverage, using a liquid or solid matrix, or friut raw material as substrates; said liquid or solid matrix was generated by saccharification of grain raw materials, containing polysaccharides.

Said alcoholic beverage includes brandy, whiskey, vodka, gin, rum, Chinese liquor, tequila, wine, rice wine, beer, shochu , sake and so on. For the brewing of Whiskey, wines, vodka, tequila, Chinese liquor, rice wine, beer, shochu and sake, the main raw materials are grain (plant), and the raw materials (such as wheat, corn, rye, barley, potato, sorghum, peas, sweet potatoes, rice, glutinous rice) need saccharification before brewing. The *S. cerevisiae* in the present invention could use the polysaccharides containing liquid or solid matrix obtained by saccharification, or the fruit raw material as substrates to produce alcoholic beverages, and can increase the carbon utilization and alcohol yield.

Said application method was an enhancement application of said *S. cerevisiae* in the production of alcoholic beverage. Said *S. cerevisiae* was inoculated into the production system in a liquid agent or a solid agent to improve the carbon source utilization and the alcohol yield.

The preparation of said liquid agent was carried out by inoculating the activated liquid seed in sterilized liquid medium at an inoculation amount of 1 to 5% (w / w) (i.e., 1 to 5 g of the liquid seed per 100 g of the liquid medium) and cultivating at 25 ∼ 35 °C for 24 ∼ 72 hours.

In one embodiment, the liquid medium was sorghum extract medium, and the sorghum extract medium preparation method comprised the following steps: 20 ∼ 200g of sorghum was crushed, and 1 to 4 times of water in mL / g was added; after cooking for 1-5h, the mixture was paste, and 10 ∼ 50 units/g glucoamylase was added and then standed at 40 ∼ 100 °C for 2 ∼ 10hours; filtrate with a sugar content of 10∼15°Bx was obtained by filtration and centrifugal.

The preparation of said solid agent was carried out by inoculating the activated liquid seed into sorghum extract medium (inoculation amount was 1 to 5% (w / w)) and solid fermentation in the environment of 25 ∼ 35 °C for 32 ∼ 72 hours.

Said application method was carried out by using *S. cerevisiae* CCTCC M 2014463 for production of fuel ethanol. Said *S. cerevisiae* was inoculated in a liquid or solid matrix composed of polysaccharide saccharified by starch raw material and cellulose raw material, and fermented to produce fuel ethanol and the carbon source utilization and ethanol yield was improved.

Said starch raw material, includes various cereal (plant) feedstocks.

Said cellulose raw material includes lignocellulosic biomass (e.g., crop stalks, chaff, bran, bagasse, trees, forestry processing wastes, grasses, etc.).

The *S. cerevisiae* of the present invention was screened from the Chinese Maotai-flavored liquor making environment. The making of Maotai-type liquor is a synchronous process of saccharification and fermentation, which makes the making system contain a variety of different carbon sources, and the system has characteristics of high temperature, high acid concentration, high ethanol concentration. The yeast screened from the making environment has characteristics of multi-carbon source utilization, tolerance to high temperature, tolerance to high acid and high ethanol concentration, and good flavor metabolism. The *S. cerevisiae* strain of the present invention is the first discovery of *S. cerevisiae* which having all the above-mentioned excellent characteristics, making it a very important application potential and value for both alcoholic beverages brewing and fuel ethanol production.

Preservation of biological materials:
*S. cerevisiae* CCTCC M 2014463, categorized by *Saccharomyces cerevisiae,* was deposited in the China Center for Type Culture Collection (CCTCC), Wuhan University, Wuhan, Hubei Province, China, on October 10, 2014.

### BRIEF DESCRIPTION OF DRAWINGS

Figure 1. Electron microscopy of *S. cerevisiae* CCTCC M 2014463.
Figure 2. Comparison of the carbon utilization capacity *of S. cerevisiae* CCTCC M 2014463 and *S. cerevisiae* S288c, a *S*. *cerevisiae* type strain; * represents the significant difference (P <0.05 analysed by one-way ANOVA).
Figure 3. Comparison of the tolerance to temperature, pH, and ethanol stress of *S*. *cerevisiae* CCTCC M 2014463 and *S. cerevisiae* S288c; * represents the significant difference (P <0.05 analysed by one-way ANOVA).
Figure 4. GC-MS spectra of the flavor metabolites of *S. cerevisiae* CCTCC M 2014463.
Figure 5. Fermentation of the strain in medium with glucose as the carbon source, where the solid icon represents *S. cerevisiae* CCTCC M 2014463 and the hollow icon represents the control strain *S. cerevisiae* S288c.
Figure 6. Fermentation of the strain in medium with glucose and maltose as a mixed carbon source, where the solid icon represents *S. cerevisiae* CCTCC M 2014463 and the hollow icon represents the control strain *S*. *cerevisiae* S288c.
Figure 7. Fermentation of the strain in medium with glucose and galactose as a mixed carbon source, where the solid icon represents *S. cerevisiae* CCTCC M 2014463 and the hollow icon represents the control strain *S*. *cerevisiae* S288c.

### EXAMPLES

Detection of flavor substances: Headspace Solid Phase Microextraction (HS-SPME) and gas chromatography-mass spectrometry (GC-MS) were used to analyze the volatile products. 8 mL of the samples were placed in a headspace bottle containing 3 g of NaCl , 10 µL of 4-methyl-2-pentanol with a concentration of 42.60 mg·L⁻¹ was added as an internal standard. The volatile products were extracted in the headspace bottle at 50 °C for 45 min and then analysed by GC-MS.

### Example 1: Carbon source assimilation of S. cerevisiae CCTCC M 2014463

A single colony was inoculated into YPD liquid medium, and cultured at 200rpm and 30 °C for 16∼20 hours to obtain liquid seed. The seed was diluted with physiological saline to OD₆₀₀ = 0.05 and transferred to Biolog YT Microplate (purchased from HUA YUE instrument company) with an inoculation amount of 100 µL, and then incubation at 28 °C for 72 hours. And then the OD₅₉₀ and OD₇₃₀ was measured. It indicated that the carbon source can be utilized but not highly utilized when the OD₅₉₀ and OD₇₃₀ were both greater than 0.2 and less than 0.8, and it indicated that the carbon source can be well used when OD₅₉₀ and OD₇₃₀ were both greater than 0.8. Two parallels were carried out in Biolog YT Microplate. The * mark on the figure represented that the P value analysed by one-way ANOVA was less than 0.05.

The results were shown in Figure 2. It showed that *S. cerevisiae* S288c could only use common carbon sources, such as glucose and sucrose, and CCTCC M 2014463 could use glucose, galactose, xylose and alfa sugar, sucrose, maltose, melibiose, loose sugar, trehalose, raffinose and other oligosaccharides. Among them, the use of galactose and maltose had been verified by shake flask experiments.

### Example 2: Tolerance of S. cerevisiae CCTCC M 2014463 to stress conditions

Comparison of environmental tolerance between *S. cerevisiae* CCTCC M 2014463 and *S*. *cerevisiae* S288c was carried out.

Tolerance to temperature: the activated strain was inoculated to YPD liquid medium with an inoculation amount of 2%, and cultivated for 48 hours at 30 °C, 37 °C and 44 °C respectively. Then, the OD₆₀₀ was measured.

Tolerance to ethanol: the activated strain was inoculated to YPD liquid medium with an inoculation amount of 2%. And then ethanol was added to 0%, 2%, 4%, 6%, 8%, 10%, 12%, 14%, respectively. And then, standing cultivation was conducted for 48 hours at 30 °C, and the OD₆₀₀ was measured.

Tolerance to acid: the activated strain was inoculated to YPD liquid medium with an inoculation amount of 2%. The pH of said YPD liquid medium was adjusted by to 5, 4.5, 4, 3.5, 3, 2.5 and 2 , respectively. After standing culturing at 30 °C for 48 hours, the OD₆₀₀ was measured.

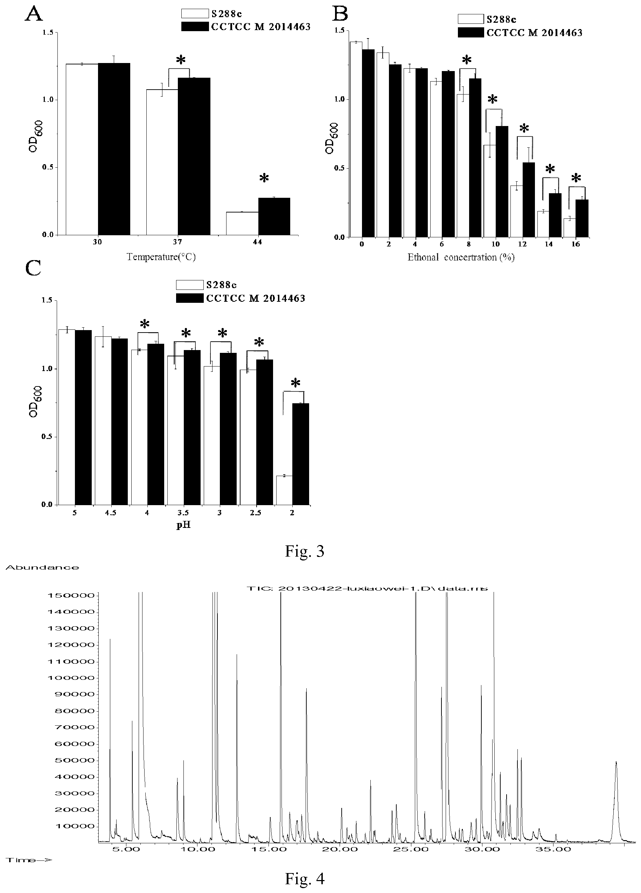

As shown in Fig. 3, *S*. *cerevisiae* CCTCC M 2014463 was resistant to high temperature (44 °C), high concentration of acid (pH 2.0) and alcohol (16%). Under pH 2, the OD of the control strain *S. cerevisiae* S288c was only 0.2, which mean *S. cerevisiae* S288c almost could not grow at pH 2. In comparison, the OD *of S. cerevisiae* CCTCC M 2014463 under pH 2 could reach 0.8, and it was 66.67% of the OD under pH 5. The data indicated that the acid resistance of CCTCC M 2014463 was stronger than that of the control strain. In addition, CCTCC M 2014463 was also more resistant to ethanol than *S. cerevisiae* S288c, and its maximum biomass at 16% alcohol concentration was 2 times that *of S. cerevisiae* S288c.

### Example 3: Flavor metabolism characteristics of S. cerevisiae CCTCC M 2014463

*S. cerevisiae* CCTCC M 2014463 could produce variety of flavor metabolites. *S*. *cerevisiae* CCTCC M 2014463 was inoculated to sorghum juice medium. Then, the broth was incubated with fermentation bolts at 30 °C for 5 days and the flavor metabolites in the fermentation broth were detected by GC-MS

As shown in Figure 4, *S*. *cerevisiae* CCTCC M 2014463 was capable of producing a variety of flavor metabolites, including acids, alcohols and esters. Said acids contained hexanoic, octanoic acid, nonanoic acid, 9-decenoic acid; said alcohols contained ethanol, isobutanol, isoamyl alcohol, butanol, hexanol, 2-nonen-1-ol, 3-metbyl-1-hexanol, 1-octanol, nonanol, 3-metbyl-1-hexanol, phenylethanol, lauryl alcohol, trans-neryl terephthalate, farnesol; said esters contained ethyl acetate, isoamyl acetate, ethyl hexanoate, ethyl 4-hexenoate, ethyl octanoate, ethyl decanoate, 2-phenylethyl acetate, benzene 2-phenylethyl acetate, isohexyl-2-phenylethyl acetate, ethyl 2,4-hexadienoate, phenylacetaldehyde and acetophenone 4-vinylxaphenols.

Said sorghum juice medium may also be replaced by YNB medium.

### Example 4: Improving carbon source utilization and ethanol production by adding S. cerevisiae CCTCC M 2014463

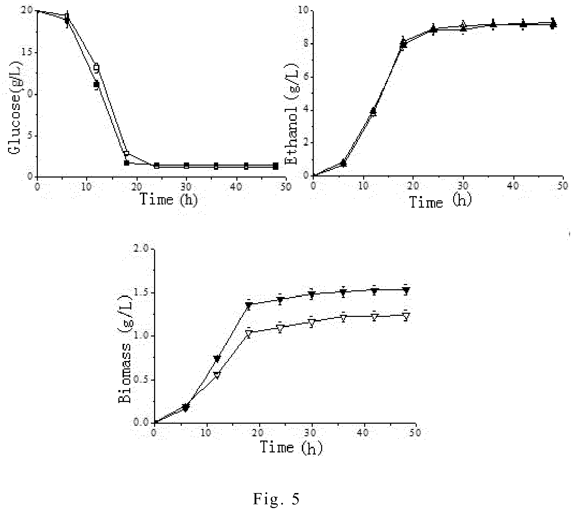

A single colony was inoculated into YPD liquid medium and cultured at 200rpm and 30 °C for 16∼20 hours. The obtained seed liquid was diluted with physiological saline to OD₆₀₀ = 1 and transferred to a single carbon source or mixed carbon source (total carbon source content was 2%). The culture was carried out at 200 rpm and 30 °C for 48 hours and sampled every 6 hours. The biomass and concentration of ethanol production and residual sugar were determinated, and the results were shown in Figure 5 to Figure 7.

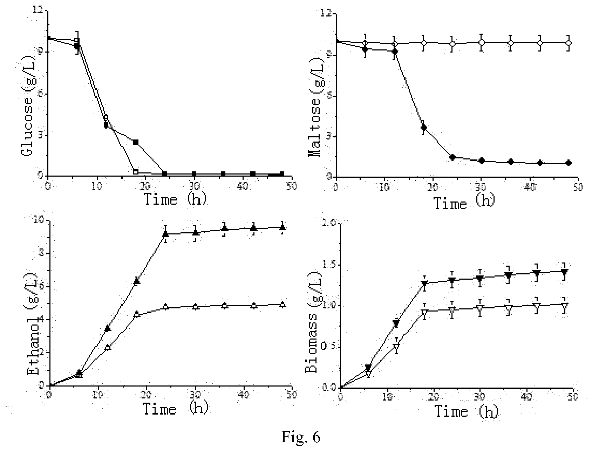

It can be seen from Figure 6 that the biomass and ethanol production of *S*. *cerevisiae* CCTCC M 2014463 were two times as high as that *of S. cerevisiae* S288c under the mixed carbon source medium containing 1% glucose and 1% maltose. In addition, the sugar curve indicated that *S. cerevisiae* CCTCC M 2014463 can use glucose and maltose simultaneously.

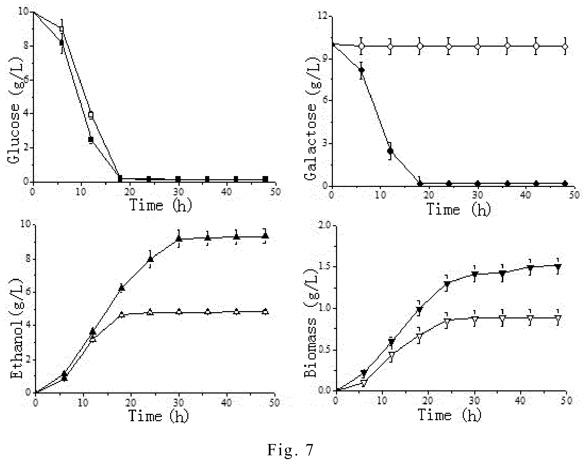

Similarly, it can be seen from Fig. 7 that the biomass and ethanol production of *S*. *cerevisiae* CCTCC M 2014463 were 2 times that *of S. cerevisiae* S288c under the mixed carbon source medium containing 1% glucose and 1% galactose. In addition, the sugar curve indicated that *S. cerevisiae* CCTCC M 2014463 can use glucose and galactose simultaneously.

The results showed that the carbon source utilization, growth and ethanol production *of S. cerevisiae* CCTCC M 2014463 were not significantly different from those of *S. cerevisiae* S288c. However, in the mixed carbon source containing maltose and glucose or galactose and glucose, *S. cerevisiae* CCTCC M 2014463 could use maltose and galactose at the early stage of its growth logarithm, and its growth curve had no secondary growth. In addition, the final ethanol production *of S. cerevisiae* CCTCC M 2014463 was twice that *of S. cerevisiae* S288c. These results demonstrated high carbon utilization and high ethanol production of *S*. *cerevisiae* CCTCC M 2014463 in mixed carbon source medium.

### Example 5: Multi-carbon-coutilizing characteristics of S. cerevisiae CCTCC M 2014463

Single colony of *S. cerevisiae* CCTCC M 2014463 or *S. cerevisiae* S288c was inoculated in YPD liquid medium, and cultured at 200 rpm and 30 °C for 16∼20 hours. The obtained seed liquid was diluted with physiological saline to OD₆₀₀ = 1 and transferred to a medium with a carbon source of 1% sucrose, 1% xylose and 1% honey disaccharide. Then culture was carried out at 200 rpm and 30 °C for 48 hours and sampled every 6 hours. The biomass and concentration of ethanol production and residual sugar were determinated. Results showed that *S. cerevisiae* CCTCC M 2014463 could use these three sugars at the early stage of its growth logarithm, and the final ethanol production was 2.6 times of that of *S. cerevisiae* S288c.
## Claims

1. A *S. cerevisiae* strain, which was deposited in China Center for Type Culture Collection (CCTCC), Wuhan University, Wuhan, Hubei Province, China, on October 10, 2014, with the accession number CCTCC M 2014463.

2. The strain of claim 1, wherein said *S. cerevisiae* can use any of the following carbon sources: glucose, galactose, xylose, alfa ketone, sucrose, maltose, honey disaccharide, loose sugar, trehalose and raffinose.

3. The strain of claim 1, wherein said *S. cerevisiae* could endure high temperature of 44 °C, acid environment of pH 2.0 and high concentration alcohol of 16%.

4. The strain of claim 1, wherein said *S. cerevisiae* is capable of producing a variety of flavor metabolites, including acids, alcohols and esters; said acids contains hexanoic, octanoic acid, nonanoic acid, 9-decenoic acid; said alcohols contains ethanol, isobutanol, isoamyl alcohol, butanol, hexanol, 2-nonen-1-ol, 3-methyl-1-hexanol, 1-octanol, nonanol, 3-metbyl-1-hexanol, phenylethanol, lauryl alcohol, trans-neryl terephthalate, farnesol; said esters contains ethyl acetate, isoamyl acetate, ethyl hexanoate, ethyl 4-hexenoate, ethyl octanoate, ethyl decanoate, 2-phenylethyl acetate, benzene 2-phenylethyl acetate, isohexyl-2-phenylethyl acetate, ethyl 2,4-hexadienoate, phenylacetaldehyde and acetophenone 4-vinylxaphenols.

5. An application method of the *S. cerevisiae* strain of claim 1, wherein said method is using *S*. *cerevisiae* CCTCC M 2014463 in the field of food.

6. An application method of the *S. cerevisiae* strain of claim 1, wherein said method is using *S*. *cerevisiae* CCTCC M 2014463 in the field of brewing.

7. The method of claim 6, wherein said method is directly using *S. cerevisiae* in the production of alcoholic beverage, using a liquid or solid matrix, or fruit raw material as substrates; said liquid or solid matrix is generated by saccharification of grain raw materials, containing polysaccharides.

8. The method of claim 6, wherein said method is an enhancement application of said *S*. *cerevisiae* in the production of alcoholic beverage; said *S. cerevisiae* is inoculated into the production system in a liquid agent or a solid agent to improve the carbon source utilization and the alcohol yield.

9. An application method of the *S. cerevisiae* strain of claim 1, wherein said method is carried out by using *S. cerevisiae* CCTCC M 2014463 for production of fuel ethanol.

10. The method of claim 9, wherein said method is inoculating the said *S. cerevisiae* to a liquid or solid matrix to produce fuel ethanol; said liquid or solid matrix is composed of polysaccharides saccharified by starch raw material and cellulose raw material

## Patentansprüche

1. *S.-cerevisiae-Stamm,* der am 10. Oktober 2014 unter der Akzessionsnummer CCTCC M 2014463 beim China Center for Type Culture Collection (CCTCC), Wuhan Universität, Wuhan, Provinz Hubei, China, hinterlegt wurde.

2. Stamm gemäß Anspruch 1, wobei das *S*. *cerevisiae* eine der folgenden Kohlenstoffquellen verwerten kann: Glucose, Galactose, Xylose, Alpha-Keton, Saccharose, Maltose, Honigdisaccharid, Turanose, Trehalose und Raffinose.

3. Stamm gemäß Anspruch 1, wobei das *S*. *cerevisiae* eine hohe Temperatur von 44 °C, eine Säureumgebung von pH 2,0 und eine hohe Alkoholkonzentration von 16% aushalten könnte.

4. Stamm gemäß Anspruch 1, wobei das *S*. *cerevisiae* eine Vielzahl von Aromametaboliten einschließlich Säuren, Alkoholen und Estern produzieren kann, die Säuren Hexansäure, Octansäure, Nonansäure, 9-Decensäure enthalten, die Alkohole Ethanol, Isobutanol, Isoamylalkohol, Butanol, Hexanol, 2-Nonen-1-ol, 3-Methyl-1-hexanol, 1-Octanol, Nonanol, 3-Methyl-1-hexanol, Phenylethanol, Laurylalkohol, trans-Nerylterephthalat, Farnesol enthalten, die Ester Ethylacetat, Isoamylacetat, Ethylhexanoat, Ethyl-4-hexenoat, Ethyloctanoat, Ethyldecanoat, 2-Phenylethylacetat, Benzol-2-phenylethylacetat, Isohexyl-2-phenylethylacetat, Ethyl-2,4-hexadienoat, Phenylacetaldehyd und Acetophenon-4-vinyloxaphenole enthalten.

5. Verfahren zur Anwendung des *S.-cerevisiae*-Stamms gemäß Anspruch 1, wobei das Verfahren darin besteht, *S. cerevisiae* CCTCC M 2014463 auf dem Gebiet der Lebensmittel zu verwenden.

6. Verfahren zur Anwendung des *S.-cerevisiae*-Stamms gemäß Anspruch 1, wobei das Verfahren darin besteht, *S*. *cerevisiae* CCTCC M 2014463 auf dem Gebiet des Brauens zu verwenden.

7. Verfahren gemäß Anspruch 6, wobei das Verfahren darin besteht, *S*. *cerevisiae* direkt bei der Produktion von alkoholischen Getränken unter Verwendung einer flüssigen oder festen Matrix oder von Fruchtrohstoff als Substrate zu verwenden, wobei die flüssige oder feste Matrix durch Verzuckerung von Kornrohstoffen, die Polysaccharide enthalten, entsteht.

8. Verfahren gemäß Anspruch 6, wobei das Verfahren eine Verstärkungsanwendung des *S*. *cerevisiae* bei der Produktion von alkoholischen Getränken ist, wobei das *S*. *cerevisiae* in einem flüssigen Mittel oder einem festen Mittel in das Produktionssystem eingeimpft wird, um die Verwertung der Kohlenstoffquellen und die Alkoholausbeute zu verbessern.

9. Verfahren zur Anwendung des *S.-cerevisiae*-Stamms gemäß Anspruch 1, wobei das Verfahren durchgeführt wird, indem man *S*. *cerevisiae* CCTCC M 2014463 für die Produktion von Kraftstoffethanol verwendet.

10. Verfahren gemäß Anspruch 9, wobei das Verfahren darin besteht, das *S*. *cerevisiae* in eine flüssige oder feste Matrix einzuimpfen, um Kraftstoffethanol zu produzieren, wobei die flüssige oder feste Matrix aus Polysacchariden besteht, die durch Stärkerohstoff und Celluloserohstoff verzuckert sind.

## Revendications

1. Souche de *S*. *cerevisiae,* qui a été déposée auprès du China Center for Type Culture Collection (CCTCC), université de Wuhan, Wuhan, province de Hubei, Chine, le 10 octobre 2014, sous le numéro d'accession CCTCC M 2014463.

2. Souche selon la revendication 1, dans laquelle ladite *S*. *cerevisiae* peut utiliser l'une quelconque des sources de carbone suivantes : le glucose, le galactose, le xylose, l'alfa-cétone, le sucrose, le maltose, le disaccharide de miel, le sucre en vrac, le tréhalose et le raffinose.

3. Souche selon la revendication 1, dans laquelle ladite *S*. *cerevisiae* pourrait supporter une température élevée de 44 °C, un environnement acide de pH 2.0 et une concentration élevée en alcool de 16 %.

4. Souche selon la revendication 1, dans laquelle ladite *S*. *cerevisiae* est capable de produire une diversité de métabolites de flaveurs, parmi lesquels des acides, des alcools et des esters ; lesdits acides contiennent de l'acide hexanoïque, de l'acide octanoïque, de l'acide nonanoïque, de l'acide 9-décènoïque ; lesdits alcools contiennent de l'éthanol, de l'isobutanol, de l'alcool isoamylique, du butanol, de l'hexanol, du 2-nonèn-1-ol, du 3-méthyl-1-hexanol, du 1-octanol, du nonanol, du 3-méthyl-1-hexanol, du phényléthanol, de l'alcool laurylique, du téréphtalate de transnéryle, du farnésol ; lesdits esters contiennent de l'acétate d'éthyle, de l'acétate d'isoamyle, de l'hexanoate d'éthyle, du 4-hexénoate d'éthyle, de l'octanoate d'éthyle, du décanoate d'éthyle, de l'acétate de 2-phényléthyle, de l'acétate de benzène-2-phényléthyle, de l'acétate d'isohexyl-2-phényléthyle, du 2,4-hexadiénoate d'éthyle, du phénylacétaldéhyde et des 4-vinylxaphénols d'acétophénone.

5. Procédé d'application de la souche de *S*. *cerevisiae* selon la revendication 1, dans lequel ledit procédé utilise la *S*. *cerevisiae* CCTCC M 2014463 dans le domaine de l'alimentation.

6. Procédé d'application de la souche de *S*. *cerevisiae* selon la revendication 1, dans lequel ledit procédé utilise la *S*. *cerevisiae* CCTCC M 2014463 dans le domaine du brassage.

7. Procédé selon la revendication 6, dans lequel ledit procédé utilise directement *S*. *cerevisiae* dans la production de boisson alcoolisée, utilise une matrice liquide ou solide ou une matière première à base de fruits en tant que substrats ; ladite matrice solide ou liquide est générée par saccharification de matières premières à base de grains, contenant des polysaccharides.

8. Procédé selon la revendication 6, dans lequel ledit procédé est une application d'amélioration de ladite *S*. *cerevisiae* dans la production de boisson alcoolisée ; ladite *S*. *cerevisiae* est inoculée dans le système de production dans un agent liquide ou un agent solide pour améliorer l'utilisation de sources de carbone et la production d'alcool.

9. Procédé d'application de la souche de *S*. *cerevisiae* selon la revendication 1, dans lequel ledit procédé est réalisé en utilisant *S*. *cerevisiae* CCTCC M 2014463 pour la production d'éthanol-carburant.

10. Procédé selon la revendication 9, dans lequel ledit procédé inocule ladite *S*. *cerevisiae* à une matrice liquide ou solide pour produire de l'éthanol-carburant ; ladite matrice liquide ou solide est composée de polysaccharides saccharifiés par une matière première à base d'amidon et une matière première à base de cellulose.


<110> Jiangnan University
<120> Saccharomyces cerevisiae capable of being co-fermented by multiple carbon sources and application thereof
<140> PCT/CN2014/093260
   <141> 2014-12-08
<160> 1
<170> PatentIn version 3.3
<210> 1
   <211> 580
   <212> DNA
   <213> 26s rDNA
<400> 1
   cacccggggt attgccttac ctacggcgag tgaagcggca aaagctcaaa tttgaaatct     60
   ggtaccttcg gtgcccgagt tgtaatttgg agagggcaac tttggggccg ttccttgtct    120
   atgttccttg gaacaggacg tcatagaggg tgagaatccc gtgtggcgag gagtgcggtt    180
   ctttgtaaag tgccttcgaa gagtcgagtt gtttgggaat gcagctctaa gtgggtggta    240
   aattccatct aaagctaaat attggcgaga gaccgatagc gaacaagtac agtgatggaa    300
   agatgaaaag aactttgaaa agagagtgaa aaagtacgtg aaattgttga aagggaaggg    360
   catttgatca gacatggtgt tttgtgccct ctgctccttg tgggtagggg aatctcgcat    420
   ttcactgggc cagcatcagt tttggtggca ggataaatcc ataggaatgt agcttgcctc    480
   ggtaagtatt atagcctgtg ggaatactgc cagctgggac tgaggactgc gacgtaagtc    540
   aaggatgctg gcataatggt tatatgccgc ccgtctgaaa                          580